# EUROPEAN PATENT APPLICATION

(11) **EP 4 585 157 A1**
(43) Date of publication of application: **16.07.2025**
(21) Application number: 24151520.4
(22) Date of filing: 12.01.2024
(51) Int. Cl.: A61B 6/08, A61B 6/00, A61B 6/58

(54) **X-RAY SYSTEM AND METHOD FOR PREPARING X-RAY EXAMINATIONS**

(71) Applicant: Koninklijke Philips N.V., 5656 AG Eindhoven (NL)
(72) Inventor: KOEPNICK, Johannes, Eindhoven (NL); LUNDT, Bernd, Eindhoven (NL); MAY, Jan Marek, 5656AG Eindhoven (NL); BYSTROV, Daniel, Eindhoven (NL)
(74) Representative: Philips Intellectual Property & Standards

(57) **Abstract**

The invention relates to an X-ray system (1) comprising an X-ray tube (2), an X-ray detection unit (3), a moving arrangement (7), a sensor unit (8) and a computing unit (9). The X-ray detection unit (3) comprises an X-ray detector (6) with an image area (13). The moving arrangement (7) is configured to adapt a position of the X-ray tube (2) and the X-ray detection unit (3) and the sensor unit (8) is configured to acquire sensor data (14) relating to a position of a body part of interest (4) of a patient (5). The computing unit (9) is configured to receive the sensor data (14), determine the position of the body part of interest (4) relative to the image area (13) based on the sensor data (14) and on system geometry information, and generate positioning commands (15) to control the moving arrangement (7) to set the X-ray tube (2) and the X-ray detection unit (3) to a preferred position with respect to the body part of interest (4) based on the determined position.

## Description

### FIELD OF THE INVENTION

The invention relates to medical imaging, in particular to X-ray imaging, more particularly to radiography and/or fluoroscopy. In particular, the invention relates to an X-ray system that automatically positions the X-ray detector with respect to a patient's position. Further, the invention relates to an X-ray detection arrangement, to a corresponding method for preparing X-ray examinations, to a computer program product and to a computer-readable medium.

### BACKGROUND OF THE INVENTION

In radiographic imaging, correctly positioning a patient's body part being imaged in an image area of an X-ray detector, i.e., aligning an X-ray tube, the body part and the X-ray detector, is required in order to achieve optimal results. Otherwise, the diagnostic quality of the acquired images may be compromised. For example, most upright chest exams require a radiographer to correctly position, e.g., to center, the patient in front of the detector. In current workflows, radiographers must accomplish this by manually positioning the patient and/or the system, which impedes workflow, takes several seconds and involves interaction with the patient.

In particular, the correct positioning is currently performed by the radiographers in interaction with the patient by giving oral guidance and/or by moving the patient into the correct position. Thus, correct patient positioning is a time consuming task, based on experience and guidelines. Of course, patients can also be immobile, which makes their positioning even more difficult.

Even when automated guidance instructions on the patient placement are used, interaction of the radiographer with the patient and with the system is still required, and the patient positioning is still time-consuming.

### SUMMARY OF THE INVENTION

It is therefore an object of the present invention to provide an X-ray system that overcomes the above-mentioned problems, in particular that provides an automatic positioning of an X-ray tube and an X-ray detector with respect to a patient. It is a further object of the present invention to provide an X-ray detection arrangement and a corresponding method for preparing X-ray examinations.

The object of the present invention is solved by the subject matter of the independent claims, wherein further embodiments are incorporated in the dependent claims.

In an aspect of the present invention, an X-ray system is provided. In this context, the X-ray system is an X-ray system for medical examinations of patients. In particular, the X-ray system is an X-ray radiography system and/or an X-ray fluoroscopy system. More particularly, the X-ray system is an X-ray system for upright examinations of a patient, e.g., for chest examinations of the patient.

The X-ray system comprises an X-ray tube, an X-ray detection unit, a moving arrangement, a sensor unit and a computing unit.

The X-ray tube provides an X-ray beam. In particular, the X-ray tube may comprise a collimator such that the X-ray tube provides a collimated X-ray beam. Said X-ray beam has a center and may have a circular, oval or rectangular shape.

The X-ray detection unit comprises an X-ray detector. Said X-ray detector may be an analog or a digital X-ray detector. The X-ray detector has an image area, wherein the image area has a center and may be rectangular.

The moving arrangement is configured to adapt a position of the X-ray tube and the X-ray detection unit. In this context, "adapting a position" comprises both changing a position, i.e., moving the X-ray tube and/or the X-ray detection unit, and keeping the position, in case the X-ray tube and/or the X-ray detection unit are already in the desired position. In particular, the moving arrangement may comprise two moving units, wherein a first moving unit is configured to adapt the position of the X-ray tube and a second moving unit is configured to adapt the position of the X-ray detection unit. Alternatively, the X-ray tube and the X-ray detection unit may be mechanically coupled to one another, e.g., both the X-ray tube and the X-ray detection unit are attached to separate ends of a C-arm, such that both the X-ray tube and the X-ray detection unit are moved by a single moving unit. Said moving units may comprise electric motors, e.g., step motors or linear motors.

The sensor unit is configured to acquire sensor data relating to a position of a body part of interest of a patient. In particular, said sensor unit may comprise non-contacting sensors. The sensor unit may be configured to acquire the sensor data in real time, i.e., with a delay that is less than 1 s, preferably less than 100 ms. The body part of interest may be, e.g., a chest of the patient, a joint of the patient, a bone of the patient or a head of the patient.

The computing unit is configured to receive the sensor data. This may be accomplished using a wired and/or a wireless connection between the sensor unit and the computing unit. Based on the sensor data and on system geometry information, the position of the body part of interest relative to the image area is determined. The sensor data may be analyzed using a conventional image processing algorithm and/or an artificial intelligence based approach, such as a trained neural network, to identify the body part of interest. The system geometry information may comprise a source-to-image distance and/or further information, in particular regarding the relative positions of the sensor unit, the X-ray tube and/or the X-ray detection unit. The position of the body part of interest relative to the image area may be calculated as a deviation of a center of the patient or of the body part of interest from the center of the image area.

Based on said determined position, positioning commands are generated to control the moving arrangement to set the X-ray tube and the X-ray detection unit to a preferred position with respect to the body part of interest. In this context, "to set" comprises both moving the X-ray tube and/or the X-ray detection unit if they are not in the preferred position yet, and keeping the position, in case the X-ray tube and/or the X-ray detection unit are already in the preferred position. Hence, achieving the preferred position of the X-ray tube and the X-ray detection unit with respect to the body part of interest can be fully automated, thereby significantly reducing the lead time for X-ray examinations. Further, said preferred position is achieved without patient contact or patient interaction, which further improves and simplifies the X-ray examination.

According to an embodiment, the X-ray detection unit further comprises an anti-scatter grid, in order to reduce scattered radiation and to improve image quality. The anti-scatter grid is arranged on the side of the X-ray detector facing the X-ray tube. Said anti-scatter grid may be an oscillating grid, blurring the contours of the grid to further improve image quality. Alternatively, the anti-scatter grid may be a fixed grid, providing a simplified mechanical design. Yet alternatively, a software correction may be applied to the image in order to reduce the effects of scattered radiation. This alternative provides the simplest mechanical design.

Alternatively, or additionally, the X-ray detection unit further comprises an automatic exposure control (AEC) chamber to control the exposure of the X-ray images. The AEC chamber is arranged on the side of the X-ray detector facing the X-ray tube. If both an anti-scatter grid and an AEC chamber are included in the X-ray detection unit, the AEC chamber may be arranged between the anti-scatter grid and the X-ray detector. Alternatively, the AEC chamber may be integrated into the X-ray detector, reducing the number of parts.

According to an embodiment, the sensor unit comprises at least one camera. Said camera may be an optical camera, such as a color camera, a black-and-white camera or an infrared camera. In order to identify the body part of interest, image recognition techniques may be used, e.g., using edge detection. Alternatively, or additionally, an artificial intelligence, such as a trained neural network may be used to identify the body part of interest. Alternatively, the camera may be a depth camera. In this context, the depth camera may be based on a time-of-flight measurement, in particular operating with visible light or infrared light. Alternatively, or additionally, the depth camera may be based on stereo triangulation, sheet-of-light triangulation or structured light. The depth camera provides a depth image, which may be also called a depth map or a range image. The depth image contains, for each pixel, information on the distance between the depth camera and a respective point in the scene, e.g., the patient. Again, the body part of interest may be identified using image recognition techniques and/or an artificial intelligence. Yet alternatively, the camera may be a combined optical and depth camera, e.g., an RGB-D camera. In this case, information from both the optical image and the depth image may be used to identify the body part of interest, making said identification more reliable and more accurate. Cameras as sensors are usually inexpensive, can provide good results for the identification of the body part of interest, and there is a wide range of software tools available to help to perform the identification of the body part of interest.

Alternatively, or additionally, the sensor unit comprises at least one Lidar sensor. With said Lidar sensor, it is also possible to obtain distance information from the Lidar sensor to respective points in the scene. Said distance information may then be analyzed, e.g., using conventional image processing algorithms and/or an artificial intelligence based approach, such as a trained neural network, to identify the body part of interest. As an alternative to the Lidar sensor, also other depth sensors or other sensors that yield sensor data that relates to the position of the body part of interest may be used. The exact choice of sensor may be made based on economic and practical aspects.

According to an embodiment, the sensor unit is attached to the X-ray tube or to a collimator of the X-ray tube. From this position, the sensor unit has the same or approximately the same view as the X-ray beam emitted by the X-ray tube, which makes the determination of the position of the body part of interest relative to the image area easier and more accurate. Further, determinations of a deviation of, e.g., the center of the body part of interest from the center of the image area, are very easy and reliable when the sensor unit is attached to the X-ray tube or to the collimator. Of course, other positions of the sensor unit are also possible, but it has to be ensured that the relative positions of the sensor unit, X-ray tube and X-ray detection unit are known.

According to an embodiment, the moving arrangement is configured to adapt a horizontal position and/or a vertical position of the X-ray tube and the X-ray detection unit. In this context, a horizontal direction and a vertical direction corresponding to the horizontal position and the vertical position, respectively, are the directions along which the X-ray detector extends. Hence, the horizontal position and/or vertical position of the X-ray detection unit and X-ray tube can be adjusted in order to achieve the preferred position. Therefore, the position adjustment of the X-ray detection unit and the X-ray tube with respect to the patient is automated in the horizontal and/or vertical direction, saving time during the preparation of the X-ray examination. Depending on the possible range of movement of the X-ray tube and the X-ray detection unit, some pre-positioning of the patient with respect to the X-ray tube and the X-ray detection unit may be necessary, however, the fine positioning is taken over by the X-ray system. If the X-ray tube and the X-ray detection unit are moved in both the horizontal direction and the vertical direction, the time savings for the examination preparation are maximized.

According to an embodiment, the X-ray detection unit is movable within a detector tray. Said detector tray preferably comprises a front cover, wherein the front cover is arranged on an X-ray tube side of the X-ray detection unit. The detector tray may further comprise side covers and/or a back cover. If the detector tray comprises the front cover, the side covers and the back cover, it completely encases the detection unit. Since the X-ray detection unit is moved within the detector tray, the detector tray is not moved, and the patient remains undisturbed. In particular, there is no movement of the detector tray itself needed, which would otherwise cause impact or distraction to the patient. In order to extend the range of possible movements, the detector tray itself may be moved, too. Then, for a coarse positioning, the detector tray is moved, and for the subsequent fine positioning, the X-ray detection unit is moved within the detector tray.

According to an embodiment, the front cover is transparent or semi-transparent. Hence, it is possible to see the X-ray detection unit and/or the contours of the X-ray detection unit through the front cover, indicating the position of the image area. This provides a visual indication to the operators of the X-ray system whether or not the X-ray detection unit has been properly positioned with respect to the body part of interest. If it is found that the X-ray detection unit has been incorrectly positioned, the X-ray examination can be delayed until the correct positioning has been achieved. Since the X-ray examination must not be repeated due to incorrect positioning, the radiation dose applied to the patient is reduced.

Alternatively, or additionally, the X-ray system further comprises visual indicators that are configured to indicate the location of the image area. With said visual indicators, operators of the X-ray system can see whether or not the X-ray detection unit has been properly positioned with respect to the body part of interest. If it is found that the X-ray detection unit has been incorrectly positioned, the X-ray examination can be delayed until the correct positioning has been achieved. Since the X-ray examination must not be repeated due to incorrect positioning, the radiation dose applied to the patient is reduced. As an example, the visual indicators may be lights, e.g., light emitting diodes (LEDs) or a strip light, arranged around a contour of the X-ray detector or X-ray detection unit. Combined with a transparent or semi-transparent front cover, the position of the image area can be very well seen. As another example, a transparent or semi-transparent front cover may be illuminated by laser light from the X-ray detection unit side of the front cover, wherein the laser light is directed to positions that indicate the position of the image area. As yet another example, lighting elements that do not affect or only slightly affect the X-ray transmission through the front cover may be integrated in the front cover and controlled to illuminate the image area and/or the contour of the image area. As a specific example, light guides may be embedded in the front cover, with first ends of the light guides being at possible locations of the contour of the image area and with corresponding light sources provided at second ends of the light guides.

According to an embodiment, the position of the body part of interest is determined as a deviation of a center of the body part of interest from a center of the image area. This is an easy and effective way of determining the position of the body part of interest. In particular, finding a center of the body part of interest is an easy task once the contours of the body part of interest have been found, e.g., by computing a weighted average. The center of the image area may also be computed like the center of the body part, but may preferably be pre-determined. Further, the deviation of the center of the body part of interest from the center of the image area may be given as only one signed number per direction, i.e., one signed number for the horizontal direction and/or one signed number for the vertical direction. Moreover, the deviation of the center of the body part of interest from the center of the image area may be directly used to control the moving arrangement to move the X-ray tube and the X-ray detection unit.

According to an embodiment, the preferred position is a position in which the body part of interest is centered with respect to the image area. That is, the preferred position is a position in which the center of the body part of interest agrees with the center of the image area when viewed perpendicular to the image area. In this case, optimal X-ray imaging of the body part of interest may be achieved.

According to an embodiment, the moving arrangement is controlled to set a position of the X-ray detection unit and/or the X-ray tube such that a central beam cast by the X-ray tube is perpendicular to the image area and hits the center of the image area. Hence, the X-ray detection unit and the X-ray tube are positioned such that the optimal imaging conditions are maintained. As an example, the X-ray tube and the X-ray detection unit may be mechanically coupled, e.g., installed on either end of a C-arm, and the moving unit(s) move the entire C-arm with the X-ray tube and the X-ray detection unit. In this case, once the position and orientation of the X-ray tube have been adjusted, the above conditions will be naturally fulfilled, since the X-ray tube and the X-ray detection unit move together. As another example, the X-ray tube and the X-ray detection unit may be moved by separate moving units. In this case, the moving arrangement may be controlled such that the moving units move the X-ray tube and the X-ray detection unit synchronously. Alternatively, the position of the X-ray tube may be adjusted first, followed by the adjustment of the position of the X-ray detection unit, or the position of the X-ray detection unit may be adjusted first, followed by the adjustment of the position of the X-ray tube.

In another aspect of the present invention, an X-ray detection arrangement is provided. Said X-ray detection arrangement comprises a detector tray, an X-ray detection unit and a moving unit. The X-ray detection unit is movable within the detector tray, and the moving unit is configured to adapt a position of the X-ray detection unit within the detector tray. In particular, said X-ray detection arrangement is the respective part of the X-ray system described above, with advantages and further embodiments corresponding to those according to the above description.

In yet another aspect of the present invention, a method for preparing X-ray examinations is provided. In particular, said method is a method for automated patient centering and/or automated centering of a body part of interest of a patient.

According to the method, sensor data relating to a position of a body part of interest of a patient is acquired by a sensor unit. Said sensor data is sent, by the sensor unit, to a computing unit. The computing unit receives the sensor data and determines the position of the body part of interest relative to an image area of an X-ray detector of an X-ray detection unit based on the sensor data and on system geometry information. Then, the computing unit generates positioning commands for setting positions of an X-ray tube and of the X-ray detection unit to a preferred position with respect to the body part of interest based on the determined position and transmits said positioning commands to a moving arrangement. The moving arrangement receives the positioning commands and sets the positions of the X-ray tube and the X-ray detection unit according to said positioning commands. In this context, setting positions refers to changing the positions, i.e., moving the X-ray tube and/or the X-ray detection unit, if the positions are not the preferred positions yet, or keeping the positions, if they are already the preferred positions. Hence, achieving the preferred position of the X-ray tube and the X-ray detection unit with respect to the body part of interest can be fully automated, thereby significantly reducing the lead time for X-ray examinations. Further, said preferred position is achieved without patient contact or patient interaction, which further improves and simplifies the X-ray examination.

Further details, embodiments and advantages of the method correspond to those X-ray system described above.

Once the positioning of the X-ray tube and the X-ray detection unit has been completed, an X-ray examination, in particular a radiography or a fluoroscopy, of the body part of interest may be performed.

According to an embodiment, the body part of interest of the patient is pre-positioned in front of the X-ray detection unit before the sensor data is acquired. For example, visual feedback and/or instructions through a voice interface may be provided to bring the patient into a desired pre-position for the diagnostic imaging examination. Said pre-positioning may be performed accurately enough so that the range of movement of the X-ray tube and the X-ray detection unit suffice to achieve the preferred position. By said pre-positioning, the range of required movement of the X-ray tube and the X-ray detection unit may be reduced compared to no pre-positioning.

According to an embodiment, the position of the X-ray tube or of the X-ray detection unit may be further fine-tuned. This fine-tuning comprises receiving fine-tuning commands and controlling the moving arrangement to adjust the position of the X-ray tube or of the X-ray detection unit according to the received fine-tuning commands. Controlling the moving arrangement may be triggered for example, by a human interaction interface (e.g. the use of a button or switch), by a gesture detection (e.g. detecting a gesture of an operator), by an interaction with a (e.g. graphical) user interface in a display through a keyboard and/or mouse, or a direct interaction with the display if employing a touch display. Then, the computing unit generates further positioning commands for setting the position of the X-ray detection unit or the X-ray tube, respectively, such that a central beam cast by the X-ray tube is perpendicular to the image area and hits the center of the image area. In particular, if the X-ray tube position has been modified, the position of the X-ray detection unit is adjusted, and if the X-ray detection unit position has been modified, the position of the X-ray tube is adjusted. Then, the computing unit transmits the further positioning commands to the moving arrangement and the moving arrangement receives the further positioning commands. According to said further positioning commands, the moving arrangement sets the position of the X-ray detection unit or the X-ray tube, respectively. Hence, even when fine-tuning of the positions is performed, the optimal positioning of the X-ray detection unit with respect to the X-ray tube is maintained.

In yet another aspect of the present invention, a computer program product is provided. The computer program product comprises instructions which, when the program is executed by a computing unit, cause the computing unit to receive sensor data relating to a position of a body part of interest of a patient, determine the position of the body part of interest relative to an image area of an X-ray detector of an X-ray detection unit based on the sensor data and on system geometry information, generate positioning commands for setting positions of an X-ray tube and of the X-ray detection unit to a preferred position with respect to the body part of interest based on the determined position, and transmit the positioning commands to a moving arrangement. Hence, the computer program product enables automatically achieving the preferred position of the X-ray tube and the X-ray detection unit with respect to the body part of interest, thereby significantly reducing the lead time for X-ray examinations. Further, said preferred position is achieved without patient contact or patient interaction, which further improves and simplifies the X-ray examination. In this context, the computing unit comprises one or several processors and may be a computer.

Details and further embodiments of the computer program product correspond to those given in the above description of the X-ray system and the method for preparing X-ray examinations.

In yet another aspect of the present invention, a computer-readable medium is provided. The computer-readable medium has stored thereon the computer program product according to the above description. Details and further embodiments of the computer-readable medium correspond to those of the computer program product as well as those given in the above description of the X-ray system and the method for preparing X-ray examinations.

It shall be understood that a preferred embodiment of the invention can also be any combination of the dependent claims with the respective independent claim. Further, it has to be noted that embodiments of the invention are described with reference to different subject matters. In particular, some embodiments are described with reference to device type claims whereas other embodiments are described with reference to method type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

These and other aspects of the invention will be apparent from and elucidated with reference to the embodiments described hereinafter.

### BRIEF DESCRIPTION OF THE DRAWINGS

In the following, preferred embodiments of the invention will be described, by way of example only, and with reference to the drawings in which:
Fig. 1 shows a schematic side view of an embodiment of an X-ray system;
Fig. 2 shows a schematic side view of another embodiment of an X-ray system;
Fig. 3 shows a schematic side view of an embodiment of an X-ray detection unit in a detector tray;
Fig. 4 shows a schematic front view of an embodiment of an X-ray detection unit in a detector tray; and
Fig. 5 shows a flowchart of an embodiment of a method for preparing X-ray examinations.

In the figures, elements which correspond to elements already described may have the same reference numerals. Examples, embodiments or optional features, whether indicated as non-limiting or not, are not to be understood as limiting the invention as claimed.

### DETAILED DESCRIPTION OF EMBODIMENTS

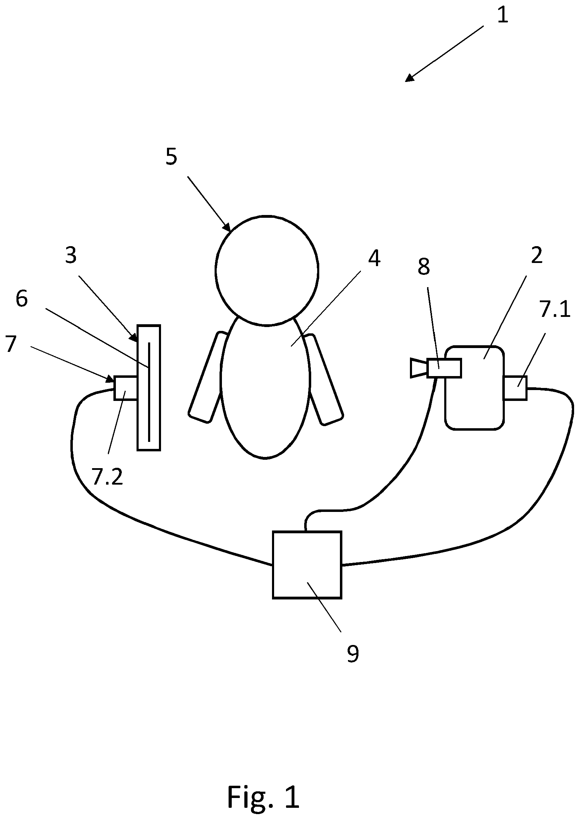

Fig. 1 shows a schematic side view of an embodiment of an X-ray system 1. In this context, the X-ray system 1 is an X-ray system 1 for medical examinations of patients. In particular, the X-ray system 1 is an X-ray radiography system and/or an X-ray fluoroscopy system.

The X-ray system 1 comprises an X-ray tube 2 and an X-ray detection unit 3. The X-ray tube 2 provides an X-ray beam. In particular, the X-ray tube may comprise a collimator such that the X-ray tube 2 provides a collimated X-ray beam. Said X-ray beam has a center and may have a circular, oval or rectangular shape. In the Figure, the X-ray tube 2 and the X-ray detection unit 3 are shown in a configuration to perform an X-ray examination of a body part of interest 4, here shown as a chest, of a patient 5. The X-ray detection unit 3 comprises an X-ray detector 6 with an image area. Said X-ray detector 6 may be an analog or a digital X-ray detector and the image area has a center and may be rectangular. The X-ray detection unit 3 may further comprise an anti-scatter grid, in order to reduce scattered radiation and to improve image quality. The anti-scatter grid is arranged on the side of the X-ray detector 6 facing the X-ray tube 2. Said anti-scatter grid may be an oscillating grid, blurring the contours of the grid to further improve image quality. Alternatively, the anti-scatter grid may be a fixed grid, providing a simplified mechanical design. Yet alternatively, a software correction may be applied to the image in order to reduce the effects of scattered radiation. This alternative provides the simplest mechanical design. Alternatively, or additionally, the X-ray detection unit 3 may further comprise an automatic exposure control (AEC) chamber to control the exposure of the X-ray images. The AEC chamber is arranged on the side of the X-ray detector 6 facing the X-ray tube 2. If both an anti-scatter grid and an AEC chamber are included in the X-ray detection unit 3, the AEC chamber may be arranged between the anti-scatter grid and the X-ray detector 6. Alternatively, the AEC chamber may be integrated into the X-ray detector 6, reducing the number of parts.

The X-ray system 1 further comprises a moving arrangement 7 that is configured to adapt a position of the X-ray tube 2 and the X-ray detection unit 3. In this context, "adapting a position" comprises both changing a position, i.e., moving the X-ray tube 2 and/or the X-ray detection unit 3, and keeping the position, in case the X-ray tube 2 and/or the X-ray detection unit 3 are already in the desired position. In the embodiment shown here, the moving arrangement 7 comprises two moving units 7.1 and 7.2, wherein the first moving unit 7.1 is configured to move the X-ray tube 2 and the second moving unit 7.2 is configured to move the X-ray detection unit 3. In particular, the moving arrangement 7 is configured to adapt a horizontal position and a vertical position of the X-ray tube 2 and the X-ray detection unit 3. In this context, the image area of the X-ray detector extends in the horizontal direction, i.e., perpendicular to the plane of projection of Fig. 1, and in the vertical direction. In alternative embodiments, the moving arrangement may be configured to adapt only the horizontal position or only the vertical position of the X-ray tube 2 and the X-ray detection unit 3.

The X-ray system 1 also comprises a sensor unit 8. The sensor unit 8 is configured to acquire sensor data relating to a position of the body part of interest 4 of the patient 5. The sensor unit 8 may comprise at least one camera, in particular an optical camera, a depth camera or a combined optical and depth camera, at least one Lidar sensor, and/or another depth sensor.

In the embodiment, the sensor unit 8 is attached to the X-ray tube 2. In particular, the sensor unit 8 may be attached to the collimator of the X-ray tube 2. From this position, the sensor unit 8 has the same or approximately the same view as the X-ray beam emitted by the X-ray tube 2, which makes a determination of the position of the body part of interest 4 relative to the image area easier and more accurate. Further, determinations of a deviation of, e.g., the center of the body part of interest 4 from the center of the image area, are very easy and reliable when the sensor unit 8 is attached to the X-ray tube 2 or to the collimator. Of course, other positions of the sensor unit 8 are also possible, but it has to be ensured that the relative positions and/or orientations of the sensor unit 8, X-ray tube 2 and X-ray detection unit 3 are known.

Further, the X-ray system 1 comprises a computing unit 9. The computing unit 9 is configured to receive the sensor data. For this, the computing unit 9 is connected to the sensor unit 8. In the Figure, a wired connection is shown, however, a wireless connection is also possible. Based on the sensor data and on system geometry information, the computing unit 9 determines the position of the body part of interest 4 relative to the image area. For this, the sensor data may be analyzed using a conventional image processing algorithm and/or an artificial intelligence based approach, such as a trained neural network, to identify the body part of interest 4. The system geometry information may comprise a source-to-image distance and/or further information, in particular regarding the relative positions of the sensor unit 8, the X-ray tube 2 and/or the X-ray detection unit 3. The position of the body part of interest 4 relative to the image area may be calculated as a deviation of a center of the body part of interest 4 from the center of the image area. Based on the determined position, positioning commands are generated to control the moving arrangement 7 to set the X-ray tube 2 and the X-ray detection unit 3 to a preferred position with respect to the body part of interest 4. In particular, the preferred position may be a position in which the body part of interest 4 is centered with respect to the image area. That is, the preferred position is a position in which the center of the body part of interest 4 coincides with the center of the image area when viewed perpendicular to the image area. In this case, optimal X-ray imaging of the body part of interest 4 may be achieved. Further, the moving arrangement 7 may be controlled to move the X-ray tube 2 such that a central beam cast by the X-ray tube 2 is perpendicular to the image area and hits the center of the image area. Hence, the X-ray tube 2 is moved such that the optimal imaging conditions are maintained. More particularly, the moving arrangement 7 is controlled such that the moving units 7.1 and 7.2 move the X-ray tube 2 and the X-ray detection unit 3 synchronously. In other words, the X-ray tube 2 follows the movement of the X-ray detection unit 3. Alternatively, the position of the X-ray tube 2 may be adjusted first, followed by the adjustment of the position of the X-ray detection unit 3, or the position of the X-ray detection unit 3 may be adjusted first, followed by the adjustment of the position of the X-ray tube 2.

With the present X-ray system 1, achieving the preferred position of the X-ray tube 2 and the X-ray detection unit 3 with respect to the body part of interest 4 can be fully automated, thereby significantly reducing the lead time for X-ray examinations. Further, said preferred position is achieved without patient contact or patient interaction, which further improves and simplifies the X-ray examination.

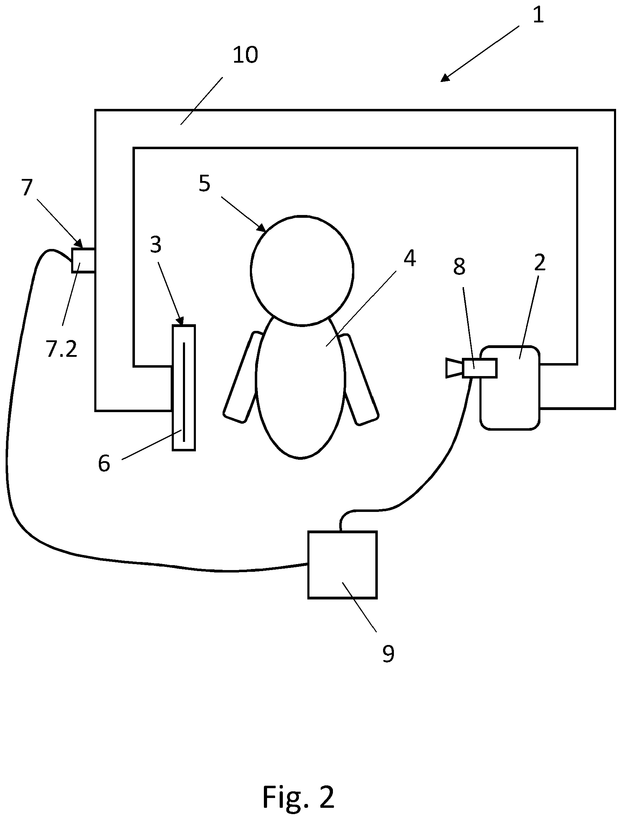

Fig.2 shows a schematic side view of another embodiment of an X-ray system 1. In this embodiment, the X-ray tube 2 and the X-ray detection unit 3 are mechanically connected to one another by a C-arm 10. In this case, the moving arrangement 7 comprises only one moving unit 7.2 that adapts a position of the entire C-arm 10 with the X-ray tube 2 and the X-ray detection unit 3. In this case, once the position and orientation of the X-ray tube 2 have been adjusted, the X-ray tube 2 will be moved such that a central beam cast by the X-ray tube 2 is perpendicular to the image area and hits the center of the image area, since the X-ray tube 2 and the X-ray detection unit 3 move together.

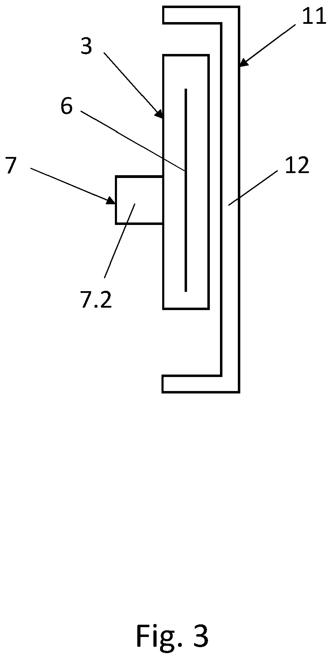

Fig.3 shows a schematic side view of an embodiment of an X-ray detection unit 3 that is movable within a detector tray 11. The detector tray 11 together with the X-ray detection unit 3 and the moving unit 7.2 that is configured to adapt a position of the X-ray detection unit 3 within the detector tray 11 may constitute an X-ray detection arrangement. The detector tray 11 may comprise a front cover 12 that is arranged on an X-ray tube 2 side of the X-ray detection unit 3. The detector tray 11 may further comprise side covers and/or a back cover. Not shown here is another embodiment in which the detector tray 11 comprises the front cover 12, the side covers and the back cover, completely encasing the detection unit 3. Since the X-ray detection unit 3 is moved within the detector tray 11, the detector tray 11 is not moved, and the patient 5 remains undisturbed. In particular, there is no movement of the detector tray 11 itself needed, which would otherwise cause impact or distraction to the patient.

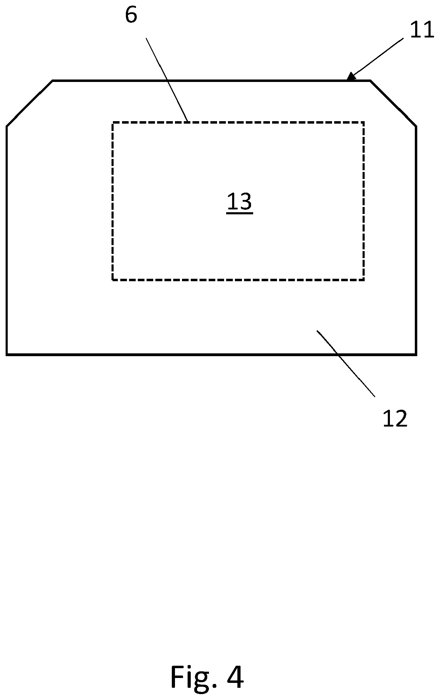

Fig.4 shows a schematic front view of an embodiment of an X-ray detection unit 3 in a detector tray 11, as seen from the X-ray tube 2 side. Here, the front cover 12 is transparent or semi-transparent such that the X-ray detector 6 and the image area 13 of the X-ray detector 6 can be seen through the front cover 12. This provides a visual indication to the operators of the X-ray system 1 whether or not the X-ray detection unit 3 has been properly positioned with respect to the body part of interest 4. If it is found that the X-ray detection unit 3 has been incorrectly positioned, the X-ray examination can be delayed until the correct positioning has been achieved. Since the X-ray examination must not be repeated due to incorrect positioning, the radiation dose applied to the patient 5 is reduced. In alternative embodiments, which are not shown here, the X-ray system 1 further comprises visual indicators that are configured to indicate the location of the image area 13. Like before, with said visual indicators, operators of the X-ray system 1 can see whether or not the X-ray detection unit 3 has been properly positioned with respect to the body part of interest 4. If it is found that the X-ray detection unit 3 has been incorrectly positioned, the X-ray examination can be delayed until the correct positioning has been achieved. Since the X-ray examination must not be repeated due to incorrect positioning, the radiation dose applied to the patient is reduced. As an example, the visual indicators may be lights, e.g., light emitting diodes (LEDs) or a strip light, arranged around a contour of the X-ray detector 6 or X-ray detection unit 3. Combined with a transparent or semi-transparent front cover 12, the position of the image area 13 can be very well seen. As another example, a transparent or semi-transparent front cover 12 may be illuminated by laser light from the X-ray detection unit 3 side of the front cover 12, wherein the laser light is directed to positions that indicate the position of the image area 13. As yet another example, lighting elements that do not affect or only slightly affect the X-ray transmission through the front cover 12 may be integrated in the front cover 12 and controlled to illuminate the image area 13 and/or the contour of the image area 13. As a specific example, light guides may be embedded in the front cover 12, with first ends of the light guides being at possible locations of the contour of the image area 13 and with corresponding light sources provided at second ends of the light guides.

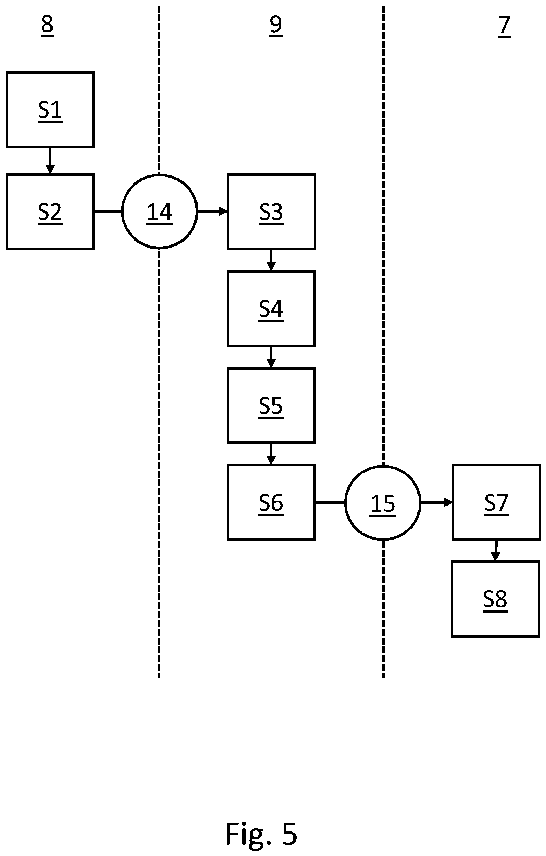

Fig.5 shows a flowchart of an embodiment of a method for preparing X-ray examinations. The sensor unit 8 acquires S1 sensor data 14 relating to the position of the body part of interest 4 of the patient 5. Then, the sensor unit 8 sends S2 the sensor data 14 to the computing unit 9. The computing unit 9 receives S3 the sensor data 14 and determines S4 the position of the body part of interest 4 relative to the image area 13 of the X-ray detector 6 of the X-ray detection unit 3 based on the sensor data 14 and on system geometry information. Then, the computing unit 9 generates S5 positioning commands 15 for setting positions of the X-ray tube 2 and of the X-ray detection unit 3 to the preferred position with respect to the body part of interest 4 based on the determined position. The computing unit 9 transmits S6 the positioning commands to the moving arrangement 7 and the moving arrangement 7 receives S7 the positioning commands 15. Finally, the moving arrangement 7 sets S8 the positions of the X-ray tube 2 and the X-ray detection unit 3 according to the positioning commands.

In an alternative embodiment which is not shown here, the body part of interest 4 of the patient 5 is pre-positioned in front of the X-ray detection unit 3 before the sensor data 14 is acquired S 1. For example, visual feedback and/or instructions through a voice interface may be provided to bring the patient 5 into a desired pre-position for the diagnostic imaging examination. Said pre-positioning may be performed accurately enough so that the range of movement of the X-ray tube 2 and the X-ray detection unit 3 suffice to achieve the preferred position. By said pre-positioning, the range of required movement of the X-ray tube 2 and the X-ray detection unit 3 may be reduced compared to no pre-positioning.

In another alternative embodiment which is not shown here, the position of the X-ray tube 2 or of the X-ray detection unit 3 may be further fine-tuned. This fine-tuning comprises receiving fine-tuning commands and controlling the moving arrangement to adjust the position of the X-ray tube or of the X-ray detection unit according to the received fine-tuning commands. Controlling the moving arrangement may be triggered for example, by a human interaction interface (e.g. the use of a button or switch), by a gesture detection (e.g. detecting a gesture of an operator), by an interaction with a (e.g. graphical) user interface in a display through a keyboard and/or mouse, or a direct interaction with the display if employing a touch display. Then, further positioning commands for setting the position of the X-ray detection unit 3 or the X-ray tube 2, respectively, are generated by the computing unit 9. These further positioning commands are generated, e.g., based on system geometry information, such that a central beam cast by the X-ray tube 2 is perpendicular to the image area 13 and hits the center of the image area 13. The further positioning commands are then transmitted by the computing unit 9 to the moving arrangement 7 and received by the moving arrangement 7. Finally, the position of the X-ray detection unit 3 or the X-ray tube 2, respectively, is set by the moving arrangement 7 according to the further positioning commands. Hence, even when fine-tuning of the positions is performed by a user, the optimal positioning of the X-ray detection unit 3 with respect to the X-ray tube 2 is maintained.

According to another example, the position of the X-ray tube 2 or of the X-ray detection unit 3 may be set to the preferred position manually, or semi-automatically (e.g. triggered by a button, or remotely controlled). Then, positioning commands for setting the position of the other part, i.e., of the X-ray detection unit 3 or of the X-ray tube 2, respectively, are generated by the computing unit 9. These positioning commands are generated, e.g., based on system geometry information, such that a central beam cast by the X-ray tube 2 is perpendicular to the image area 13 and hits the center of the image area 13. The positioning commands are then transmitted by the computing unit 9 to the moving arrangement 7 and received by the moving arrangement 7. Finally, the position of the X-ray detection unit 3 or the X-ray tube 2, respectively, is set by the moving arrangement 7 according to the further positioning commands. Hence, even when the position of the X-ray tube 2 or of the X-ray detection unit 3 is set manually or semi-automatically, the optimal positioning of the X-ray detection unit 3 with respect to the X-ray tube 2 is maintained.

Not shown here is a computer program product that comprises instructions which, when the program is executed by the computing unit 9, cause the computing unit 9 to receive the sensor data 14 relating to the position of the body part of interest 4 of the patient 5, determine the position of the body part of interest 4 relative to the image area 13 of the X-ray detector 6 of the X-ray detection unit 3 based on the sensor data 14 and on system geometry information, generate positioning commands 15 for moving the X-ray tube 2 and the X-ray detection unit 3 to the preferred position with respect to the body part of interest 4 based on the determined position, and transmit the positioning commands 15 to the moving arrangement 7. Hence, the computer program product enables automatically achieving the preferred position of the X-ray tube 2 and the X-ray detection unit 3 with respect to the body part of interest 4, thereby significantly reducing the lead time for X-ray examinations. Further, said preferred position is achieved without patient contact or patient interaction, which further improves and simplifies the X-ray examination. In this context, the computing unit 9 comprises one or several processors and may be a computer.

Also not shown here is a computer-readable medium that has stored thereon the computer program product according to the above description.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. Any reference signs in the claims should not be construed as limiting the scope.

### LIST OF REFERENCE SIGNS:

- 1: X-ray system
- 2: X-ray tube
- 3: X-ray detection unit
- 4: body part of interest
- 5: patient
- 6: X-ray detector
- 7: moving arrangement
- 7.1: first moving unit
- 7.2: second moving unit
- 8: sensor unit
- 9: computing unit
- 10: C-arm
- 11: detector tray
- 12: front cover
- 13: image area
- 14: sensor data
- 15: positioning commands
- S1: acquiring sensor data
- S2: sending sensor data
- S3: receiving sensor data
- S4: determining the position
- S5: generating positioning commands
- S6: transmitting positioning commands
- S7: receiving positioning commands
- S8: setting the positions of the X-ray tube and the X-ray detection unit

## Claims

1. An X-ray system (1) comprising
an X-ray tube (2);
an X-ray detection unit (3) comprising an X-ray detector (6) with an image area (13);
a moving arrangement (7) configured to adapt a position of the X-ray tube (2) and the X-ray detection unit (3);
a sensor unit (8) configured to acquire sensor data (14) relating to a position of a body part of interest (4) of a patient (5); and
a computing unit (9) configured to receive the sensor data (14), determine the position of the body part of interest (4) relative to the image area (13) based on the sensor data (14) and on system geometry information, and generate positioning commands (15) to control the moving arrangement (7) to set the X-ray tube (2) and the X-ray detection unit (3) to a preferred position with respect to the body part of interest (4) based on the determined position.

2. The X-ray system (1) according to claim 1, wherein the sensor unit (8) comprises at least one camera, in particular an optical camera, a depth camera or a combined optical and depth camera and/or at least one Lidar sensor.

3. The X-ray system (1) according to claim 1 or 2, wherein the sensor unit (8) is attached to the X-ray tube (2) or to a collimator of the X-ray tube (2).

4. The X-ray system (1) according to any one of claims 1 to 3, wherein the moving arrangement (7) is configured to adapt a horizontal position and/or a vertical position of the X-ray tube (2) and of the X-ray detection unit (3).

5. The X-ray system (1) according to any one of claims 1 to 4, wherein the X-ray detection unit (3) is movable within a detector tray (11), wherein the detector tray (11) preferably comprises a front cover (12).

6. The X-ray system (1) according to claim 5, wherein
the front cover (12) is transparent or semi-transparent and/or
the X-ray system (1) further comprises visual indicators configured to indicate the location of the image area (13).

7. The X-ray system (1) according to any one of claims 1 to 6, wherein the position of the body part of interest (4) is determined as a deviation of a center of the body part of interest (4) from a center of the image area (13).

8. The X-ray system (1) according to any one of claims 1 to 7, wherein the preferred position is a position in which the body part of interest (4) is centered with respect to the image area (13).

9. The X-ray system (1) according to any one of claims 1 to 8, wherein the moving arrangement (7) is controlled to set a position of the X-ray detection unit (3) and/or of the X-ray tube (2) such that a central beam cast by the X-ray tube (2) is perpendicular to the image area (13) and hits the center of the image area (13).

10. An X-ray detection arrangement comprising
a detector tray (11);
an X-ray detection unit (3) movable within the detector tray (11); and
a moving unit (7.2) configured to adapt a position of the X-ray detection unit (3) within the detector tray (11).

11. A method for preparing X-ray examinations comprising:
acquiring (S1), by a sensor unit (8), sensor data (14) relating to a position of a body part of interest (4) of a patient (5);
sending (S2), by the sensor unit (8), the sensor data (14) to a computing unit (9);
receiving (S3), by the computing unit (9), the sensor data (14);
determining (S4), by the computing unit (9), the position of the body part of interest (4) relative to an image area (13) of an X-ray detector (6) of an X-ray detection unit (3) based on the sensor data (14) and on system geometry information;
generating (S5), by the computing unit (9), positioning commands (15) for setting positions of an X-ray tube (2) and of the X-ray detection unit (3) to a preferred position with respect to the body part of interest (4) based on the determined position;
transmitting (S6), by the computing unit (9), the positioning commands (15) to a moving arrangement (7);
receiving (S7), by the moving arrangement (7), the positioning commands (15); and
setting (S8), by the moving arrangement (7), the positions of the X-ray tube (2) and the X-ray detection unit (3) according to the positioning commands (15).

12. The method according to claim 11, further comprising, before the step of acquiring (S1) sensor data (14): pre-positioning the body part of interest (4) of the patient (5) in front of the X-ray detection unit (3).

13. The method according to claim 11 or 12, further comprising:
fine-tuning the position of the X-ray tube (2) or of the X-ray detection unit (3), comprising receiving fine-tuning commands and controlling the moving arrangement (7) to adjust the position of the X-ray tube (2) or of the X-ray detection unit (3) according to the received fine-tuning commands;
generating, by the computing unit (9), further positioning commands for setting the position of the X-ray detection unit (3) or the X-ray tube (2), respectively, such that a central beam cast by the X-ray tube (2) is perpendicular to the image area (13) and hits the center of the image area (13);
transmitting, by the computing unit (9), the further positioning commands to the moving arrangement (7);
receiving, by the moving arrangement (7), the further positioning commands; and
setting, by the moving arrangement (7), the position of the X-ray detection unit (3) or the X-ray tube (2), respectively, according to the further positioning commands.

14. A computer program product comprising instructions which, when the program is executed by a computing unit (9), cause the computing unit (9) to carry out the steps of:
receiving sensor data (14) relating to a position of a body part of interest (4) of a patient (5);
determining the position of the body part of interest (4) relative to an image area (13) of an X-ray detector (6) of an X-ray detection unit (3) based on the sensor data (14) and on system geometry information;
generating positioning commands (15) for setting positions of an X-ray tube (2) and of the X-ray detection unit (3) to a preferred position with respect to the body part of interest (4) based on the determined position; and
transmitting the positioning commands (15) to a moving arrangement (7).

15. A computer-readable medium having stored thereon the computer program product of claim 14.
